# EUROPÄISCHE PATENTSCHRIFT

(11) **EP 0 737 664 B1**
(45) Veröffentlichungstag und Bekanntmachung des Hinweises auf die Patenterteilung: **28.01.1998**
(21) Anmeldenummer: 96102399.1
(22) Anmeldetag: 17.02.1996
(51) Int. Cl.: C07C 67/08, C07C 69/26, C07C 29/149, C07C 31/125

(54) **Verfahren zum Erzeugen von Wachsester und Hydrieren des Wachsesters zu Fettalkohol**
Process for the production of wax ester and hydrogenation of the wax ester to fatty alcohol
Procédé de production d'ester cireux et d'hydrogénation de l'ester cireux en alcool gras

(30) Priorität: 11.04.1995 DE 19513207
(43) Veröffentlichungstag der Anmeldung: 16.10.1996
(73) Patentinhaber: METALLGESELLSCHAFT AG, 60323 Frankfurt am Main (DE)
(72) Erfinder: Buchold, Henning, Dr., D-63452 Hanau (DE); Gärtner, Fritz-Jürgen, Dr., D-64404 Bickenbach (DE); Mallok, Gert, D-63454 Hanau (DE); Schlichting, Eberhard, Dr., D-61273 Wehrheim (DE); Stönner, Hans-Martin, D-65760 Eschborn (DE)

(56) Entgegenhaltungen:
- WO-A-90/08121
- WO-A-90/12775
- DE-A- 2 853 990

## Beschreibung

Die Erfindung betrifft ein Verfahren zum Erzeugen von Fettalkohol aus einem mindestens eine Fettsäure enthaltenden flüssigen Ausgangsgemisch, wobei die Fettsäure pro Molekül 6 bis 30 C-Atome enthält.

Im deutschen Patent 28 53 990 und im dazu korrespondierenden US-Patent 4 259 536 wird die Herstellung von Fettalkoholen durch katalytische Hydrierung von Fettsäuren in der flüssigen Phase beschrieben. Aus DE-A-39 13 387 ist es bekannt, flüssige Fettsäure-Methylester katalytisch zu hydrieren und ein Gemisch aus Fettalkoholen und Methanol zu erzeugen.

Der Erfindung liegt die Aufgabe zugrunde, ausgehend von Fettsäuren Fettalkohole nach einem Verfahren zu erzeugen, das besonders kostengünstig ist, hohe Ausbeuten ermöglicht und das sich besonders auch für Großanlagen eignet. Erfindungsgemäß gelingt dies dadurch, daß das flüssige Ausgangsgemisch pro Mol Fettsäure 1,0 bis 1,5 Mol Fettalkohol aufweist, wobei der Fettalkohol pro Molekül 6 bis 30 C-Atome enthält, daß man aus dem flüssigen Ausgangsgemisch bei Temperaturen im Bereich von etwa 120 bis 320°C ein zu mindestens 50 Gew.-% aus Wachsester bestehendes flüssiges Zwischenprodukt erzeugt, wobei man das flüssige Ausgangsgemisch durch mindestens eine Rührzone leitet und wachsesterhaltiges flüssiges Gemisch in mindestens eine Sprühzone hinein versprüht, in der Sprühzone eine wasserdampfhaltige Atmosphäre erzeugt und die wasserdampfhaltige Atmosphäre mindestens teilweise entfernt, daß man das zu mindestens 50 Gew.-% aus Wachsester bestehende Zwischenprodukt hydriert und ein zu mindestens 75 Gew.-% aus Fettalkohol bestehendes Rohprodukt erzeugt, daß man aus dem Rohprodukt einen fettalkoholhaltigen Teilstrom abtrennt und den Teilstrom dem flüssigen Ausgangsgemisch zugibt.

Das Verfahren verzichtet bewußt auf die Herstellung von Methylester als Zwischenprodukt, wodurch die Hydrierung von Methylester entfällt. Es hat sich nämlich gezeigt, daß die Veresterung der Fettsäuren mit Methanol zu Methylester ziemlich aufwendig ist, weil Nebenprodukte, z. B. Dimethylether, in Kauf genommen werden müssen und weil ferner hierbei auch mit einem Katalysator gearbeitet werden muß. Demgegenüber erzeugt man beim erfindungsgemäßen Verfahren zunächst ein wachsesterreiches Zwischenprodukt ohne einen störenden Gehalt an Nebenprodukten. Das wachsesterreiche Zwischenprodukt kann man mit oder ohne Katalysator herstellen, wobei das Arbeiten ohne Katalysator die kostengünstigste Variante ist. Wenn ein Katalysator verwendet wird, kann es sich hierbei z.B. um einen Zeolithen handeln, wie das in der Zeitschrift JAOCS, Band 69, Nr. 11 (November 1992), Seiten 1150 bis 1153 beschrieben ist.

Beim Verfahren der Erfindung wird das zu mindestens 50 Gew.-% und vorzugsweise zu mindestens 90 Gew.-% aus Wachsester bestehende Zwischenprodukt hydriert, um die gewünschten Fettalkohole zu erzeugen. Die Hydrierung dieses Zwischenprodukts läßt sich ohne Schwierigkeiten durchführen, wobei keine oder kaum Nebenprodukte (z. B. Methanol) entstehen.

Theoretisch verläuft das erfindungsgemäße Verfahren in folgender Weise:
1 Mol Fettsäure + 1 Mol Fettalkohol ergeben 1 Mol Wachsester + 1 Mol Wasser und
1 Mol Wachsester + 2 Mol H₂ ergeben 2 Mol Fettalkohol als Rohprodukt, wobei man aus dem Rohprodukt 1 Mol Fettalkohol abzweigt und zum Ausgangsgemisch zurückführt.

Das wachsesterreiche Zwischenprodukt kann kontinuierlich oder chargenweise hergestellt werden. Für die kontinuierliche Arbeitsweise empfiehlt es sich, die Fettsäure und den Fettalkohol in einer ersten Reaktionszone zu mischen, wobei ein wachsesterhaltiges Gemisch entsteht, das man durch mindestens eine weitere Reaktionszone leitet, in welcher man das Gemisch bewegt, so daS ein Rühreffekt entsteht. Ferner wird das Gemisch in mindestens einer Sprühzone versprüht.

Für die Bildung des Wachsesters ist es wichtig, daS der bei der Veresterung entstehende Wasserdampf mindestens teilweise aus dem Reaktionsgemisch entfernt wird. Dies kann auf verschiedene Weise erfolgen, z.B. dadurch, daS man die wasserdampfhaltige Atmosphäre über dem Gemisch absaugt. Eine weitere Möglichkeit besteht darin, durch das Gemisch ein Inertgas, z.B. Stickstoff, hindurchzuleiten. Das Inertgas erzeugt einen Strippeffekt, wobei das Inertgas Wassermoleküle sowohl aus dem flüssigen Gemisch als auch aus der Sprühzone mitnimmt.

Es empfiehlt sich, das Gemisch in die Sprühzone hinein fein zu verdüsen, wobei man Tröpfchendurchmesser vorzugsweise im Bereich von 0,01 bis 5,0 mm ausbildet.

Das Verhältnis von Fettsäure zu Fettalkohol im Ausgangsgemisch kann in weitem Bereich variieren. Wenn man ein wachsesterreiches Produkt erzeugen will, das nur noch geringe Reste an Fettsäure und Fettalkohol aufweist, wird man Fettsäure und Fettalkohol im Molverhältnis 1 : 1 vorlegen. Wenn es darauf ankommt, ein möglichst säurefreies Wachsester-Zwischenprodukt herzustellen, empfiehlt es sich, mit einem Überschuß an Fettalkohol zu beginnen. Auf diese Weise ist es ohne weiteres möglich, Wachsester mit einer Säurezahl kleiner 1 oder auch kleiner 0,1 herzustellen. Die Säurezahl wird wie üblich gemessen in mg KOH zum Neutralisieren der restlichen Säure pro Gramm der untersuchten Probe.

Die Hydrierung des zu mindestens 50 Gew.-% und vorzugsweise zu mindestens 90 Gew.-% aus Wachsester bestehenden Zwischenprodukts kann auf verschiedene Weise erfolgen. Eine erste Möglichkeit besteht darin, das Zwischenprodukt mit Wasserstoff über einen im Festbett angeordneten Katalysator fließen zu lassen, wobei die Temperaturen üblicherweise im Bereich von 100 bis 300°C liegen und bei einem Druck von 20 bis 400 bar gearbeitet wird. Hierbei ist es zusätzlich von Vorteil, daß man diese Hydrierung adiabatisch durchführen kann, d.h., daß man im Schachtreaktor auf Kühleinrichtungen verzichtet.

Eine weitere Möglichkeit der Hydrierung des wachsesterreichen Zwischenprodukts besteht darin, daß man in der flüssigen Phase unter Zugabe von Wasserstoff und Zumischen eines feinkörnigen Katalysators arbeitet. Ein hierfür geeigneter Hydrierreaktor ist im deutschen Patent 28 53 990 beschrieben.

Ausgestaltungsmöglichkeiten des Verfahrens werden mit Hilfe der Zeichnung erläutert. Es zeigt:
- Fig. 1: eine einstufige Verfahrensweise zur Herstellung des wachsesterreichen Zwischenprodukts,
- Fig. 2: eine dreistufige Verfahrensweise zur Herstellung des wachsesterreichen Zwischenprodukts,
- Fig. 3: eine erste Variante der Hydrierung des Zwischenprodukts und
- Fig. 4: eine zweite Variante der Hydrierung des Zwischenprodukts.

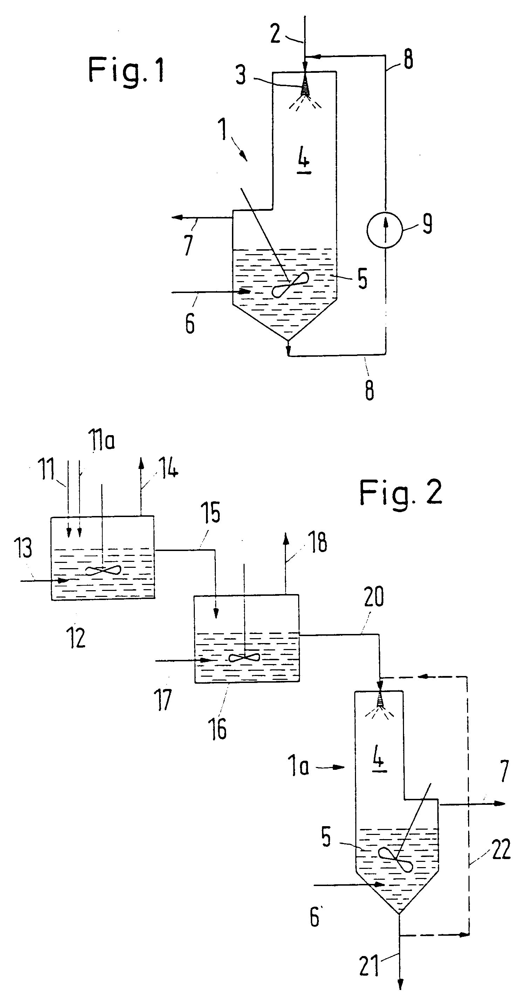

Zunächst werden die Varianten zur Herstellung des wachsesterreichen Zwischenprodukts mit Hilfe der Fig. 1 und 2 erläutert. Beim Verfahren der Fig. 1 werden einem Reaktor (1) durch die Leitung (2) gemeinsam Fettsäure und Fettalkohol zugeführt und das Gemisch wird durch eine Düse (3) in feine Tröpfchen zerteilt in die Sprühzone (4) des Reaktors (1) hinein versprüht. Unterhalb der Sprühzone (4) befindet sich im Reaktor (1) eine Reaktionszone (5), wo das Gemisch aus Fettsäure, Fettalkohol und gebildetem Wachsester ständig intensiv gerührt wird. In das Gemisch wird durch die Leitung (6) Inertgas, z.B. Stickstoff, eingeleitet. Bei der Umsetzung von Fettsäure mit Fettalkohol bei den im Reaktor herrschenden Temperaturen im Bereich von etwa 120 bis 320°C entsteht Wasserdampf, der vor allem durch das Versprühen wirksam aus dem Reaktionsgemisch abgetrennt wird. Der gebildete Wasserdampf wird zusammen mit Inertgas durch die Leitung (7) aus dem Reaktor (1) abgeführt, z.B. durch Absaugen. Falls Ausgangsmaterial und Wachsester-Produkt mitgerissen werden, kann man dies in an sich bekannter, nicht dargestellter Weise durch Kondensation wiedergewinnen und zurück in den Reaktor (1) leiten.

Die Wachsester enthaltende Flüssigkeit, die sich in der Reaktionszone (5) sammelt, wird für eine gewisse Zeit im Kreis geführt und durch die Leitung (8) unter der Wirkung der Pumpe (9) auf den Kopf des Reaktors (1) gegeben und erneut durch die Düse (3) versprüht. Bei chargenweisem Betrieb des Reaktors (1) liegen die Verweilzeiten im Reaktor bei 1 bis 10 Stunden, wobei die Gesamtmenge der Flüssigkeit 1 bis 20 mal pro Stunde versprüht wird.

Das Verfahren der Fig. 2 eignet sich besonders für kontinuierliches Arbeiten. Man kann hierbei ebenso wie beim Verfahren der Fig. 1 auch ohne Katalysator arbeiten. Gemäß Fig. 2 gibt man durch die Leitung (11) Fettsäure und durch die Leitung (11a) eine fettalkoholreiche Flüssigkeit in einen ersten Reaktionsbehälter (12), in welchem das Gemisch intensiv gerührt wird. Während des Rührens führt man durch die Leitung (13) Inertgas, z.B. Stickstoff, zu. Wasserdampfhaltiges Inertgas zieht man durch die Leitung (14) ab. Im ersten Reaktionsbehälter (12) erfolgt die Umsetzung nur teilweise, so daß man in der Leitung (15) eine Fettsäure, Fettalkohol und Wachsester enthaltende Flüssigkeit abführt, die man in einem zweiten Reaktionsbehälter (16) weiter behandelt. Dieser zweite Behälter (16) arbeitet im Prinzip genauso wie der erste Reaktionsbehälter (12), er weist ebenfalls eine Inertgaszufuhr (17) und eine Ableitung (18) für wasserdampfhaltiges Gas auf.

In der Leitung (20) zieht man aus dem zweiten Reaktionsbehälter (16) eine Flüssigkeit ab, die gegenüber der Flüssigkeit der Leitung (15) einen erhöhten Anteil an Wachsester und daneben noch Reste an Fettsäure und Fettalkohol enthält. Diese Flüssigkeit gibt man einem Reaktor (1a) auf, der, wie bereits zusammen mit Fig. 1 erläutert, eine Sprühzone (4) und eine Reaktionszone (5) aufweist. Inertgas wird durch die Leitung (6) zugeführt und wasserdampfhaltiges Gas führt man in der Leitung (7) ab. Wachsesterreiches Zwischenprodukt fällt in der Leitung (21) an. Falls erforderlich, kann man den Reaktor (1a) ebenfalls mit einem Flüssigkeitskreislauf betreiben, wobei man einen Teil der Flüssigkeit der Leitung (21) durch die gestrichelt eingezeichnete Leitung (22) zum Kopf des Reaktors (1a) zurückführt. Die Gesamt-Verweilzeit beim kontinuierlichen Verfahren gemäß Fig. 2 ist üblicherweise länger als die Verweilzeit beim chargenweisen Verfahren der Fig. 1.

Das Verfahren kann abweichend von Fig. 2 auch nur mit einem Reaktionsbehälter oder aber auch mit mehr als zwei Reaktionsbehältern betrieben werden. Auch kann man mehr als einen mit Sprüheinrichtung versehenen Reaktor einsetzen.

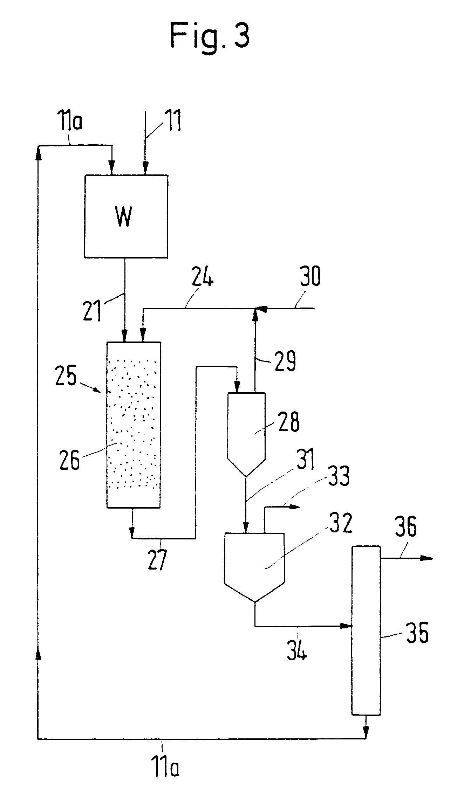

In der Verfahrensführung gemäß Fig. 3 erfolgt die Herstellung des wachsesterreichen Zwischenprodukts im Anlagenteil (W) etwa so, wie es zusammen mit Fig. 2 beschrieben wurde. Abweichend davon kann das Zwischenprodukt z.B. aber auch gemäß Fig. 1 erzeugt werden. Beim Verfahren der Fig. 3 führt man in den Anlagenteil (W) durch die Leitung (11) Fettsäure oder üblicherweise ein Fettsäuregemisch (C₆ bis C₃₀) und setzt es mit einem fettalkoholreichen Strom aus der Leitung (11a) um. Das Zwischenprodukt, das zu mindestens 50 Gew.-% und vorzugsweise zu mindestens 90 Gew.-% aus Wachsester besteht, wird in der Leitung (21) abgezogen und zusammen mit Wasserstoff, der in der Leitung (24) herangeführt wird, im Schachtreaktor (25) hydriert. Der Schachtreaktor (25) enthält eine Schüttung (26) aus körnigem Katalysator, z.B. auf Kupfer-Basis, der an sich bekannt ist. Die Korngrößen des Katalysators liegen üblicherweise im Bereich von 1 bis 30 mm. Im Reaktor (25) herrschen Temperaturen im Bereich von 100 bis 300°C und ein Druck im Bereich von 20 bis 400 bar. Hydriertes Rohprodukt zusammen mit nicht umgesetztem Wasserstoff zieht man in der Leitung (27) ab und gibt es in einen Heißabscheidebehälter (28), wo unter leichter Entspannung der Wasserstoff ausgast und in der Leitung (29) abgezogen wird. Zusammen mit frischem Wasserstoff aus der Leitung (30) wird der Wasserstoff in der Leitung (24) zurück zum Reaktor (25) geführt.

Ein zu mindestens 75 Gew.-% aus Fettalkohol bestehendes Rohprodukt zieht man aus dem Abscheider (28) in der Leitung (31) ab und führt es in einen Entspannungsbehälter (32), aus welchem man in der Leitung (33) eine kleine Menge an Wasserdampf abzieht, der durch Nebenreaktionen entstanden ist. Das verbleibende Rohprodukt wird in der Leitung (34) einer Destillationskolonne (35) aufgegeben, wobei man in der Leitung (36) Fettalkohol als Produkt abzieht. Das Sumpfprodukt der Destillationskolonne (35), das aus einem Gemisch von Fettalkoholen und Wachsester besteht, führt man in der Leitung (11a) zurück zum Anlagenteil (W).

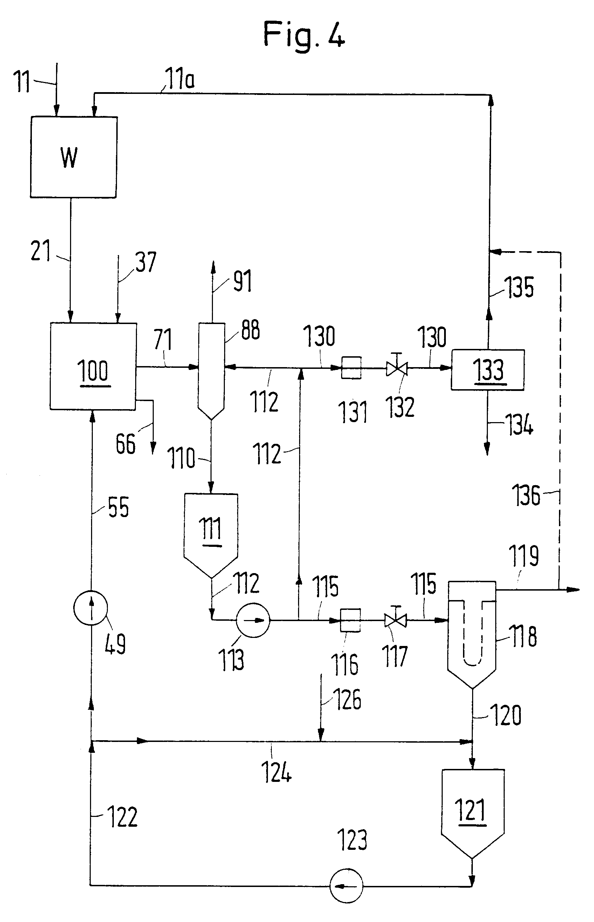

In der Verfahrensvariante der Fig. 4 wird in bereits beschriebener Weise im Anlagenteil (W) aus Fettsäure oder üblicherweise einem Fettsäuregemisch (C₆ bis C₃₀) mit in der Leitung (11a) herangeführtem Fettalkohol-Wachsester-Gemisch das wachsesterreiche Zwischenprodukt (Leitung 21) erzeugt. Dieses Zwischenprodukt wird unter Zugabe von Wasserstoff aus der Leitung (37) in flüssiger Phase in der Hydrierung (100) katalytisch bei Temperaturen im Bereich von 100 bis 400°C und Drücken im Bereich von 20 bis 400 bar hydriert. Einzelheiten dieser Hydrierung sind in den deutschen Patenten 28 53 990 und 43 04 420 beschrieben.

Der Hydrierung (100) führt man über eine Hochdruck-Dosierpumpe (49) durch die Leitung (55) eine Dispersion zu, die frischen und gebrauchten feinkörnigen Katalysator enthält. Ein Rohprodukt, das Fettalkohole und gebrauchten Katalysator enthält, wird in der Leitung (71) abgezogen und zunächst in einen Entspannungsbehälter (88) geleitet. In geringer Menge entstehendes Reaktionswasser wird über die Leitung (66) abgeschieden. Wasserstoffhaltiges Entspannungsgas zieht man in der Leitung (91) ab und kann es in nicht dargestellter Weise ganz oder teilweise in die Hydrierung (100) zurückführen. Das entspannte, flüssige Produkt, das im Behälter (88) anfällt, gelangt in der Leitung (110) zu einem Zwischenbehälter (111). Durch die Kreislaufleitung (112) und die Kreislaufpumpe (113) wird der Produktstrom teilweise zum Entspannungsbehälter (88) zurückgeführt.

Einen ersten Teilstrom des Produktstroms der Kreislaufleitung (112) zieht man in der Leitung (115) ab, führt ihn durch einen Durchflußmesser (116) und ein Regelventil (117) und gibt ihn dem Filter (118) - z. B. einem Schlauchfilter - auf.

Im Filter (118) wird ein fettalkoholreiches, feststofffreies Produkt gewonnen, das man in der Leitung (119) abzieht. Dieses Produkt kann noch einer nicht dargestellten Feinstreinigung unterzogen werden.

Im Filter (118) fällt eine gebrauchten Katalysator enthaltende Dispersion an, die man durch die Leitung (120) zunächst zu einem Zwischenbehälter (121) führt, bevor man sie durch die Leitung (122) über die Vordruckpumpe (123) teilweise in die Leitung (55) einspeist. Zweckmäßigerweise leitet man einen Teil der Dispersion durch die Leitung (124) kontinuierlich im Kreislauf zurück zum Zwischenbehälter (121). Frischer Katalysator kommt aus der Leitung (126) und wird in dosierter Menge der Dispersion der Leitung (124) zugemischt. Auf diese Weise wird eine homogene Verteilung des frischen Katalysators in der Dispersion erreicht.

Aus der Kreislaufleitung (112) wird ein zweiter Teilstrom des flüssigen Produkts durch die Leitung (130) abgezweigt und über einen Durchflußmesser (131) und ein Regelventil (132) einem Dekanter (133) zugeführt. An die Stelle des Dekanters (133) kann z.B. auch eine Zentrifuge treten. Der Dekanter (133) gibt in der Leitung (134) eine katalysatorreiche Phase ab, die üblicherweise zu 50 bis 80 Gew.-% aus Feststoff besteht. Diese Phase kann problemlos deponiert werden, auch ist ihre Wiederaufarbeitung möglich. Der im Dekanter (133) anfallende Fettalkohol wird durch die Leitung (135) abgezogen und durch die Leitung (11a) in den Anlagenteil (W) zurückgeführt. Falls in (W) mehr Fettalkohol gebraucht wird, zweigt man in der gestrichelten Leitung (136) einen Teil des Produkts der Leitung (119) ab und gibt es dem Fettalkohol der Leitung (135) zu. Die Produktströme, die durch die Leitungen (115) und (130) aus der Kreislaufleitung (112) abgezogen werden, sind variabel und üblicherweise unterschiedlich groß, das Mengenverhältnis liegt im Bereich von 100 : 1 bis 5 : 1.

Änderungen des Mengenverhältnisses führt man durch Betätigen der Regelventile (117) und (132) herbei.

### Beispiele

Man arbeitet im Labormaßstab und stellt zunächst Wachsester gemäß Figur 1 chargenweise wie folgt her: Einem Reaktor (1) mit elektrisch beheizbarem Mantel werden 208 g eines Fettsäuregemisches (C₈ bis C₁₈) mit mittlerem Molekulargewicht der Fettsäuren von 208 zusammen mit 1,1 mol C₁₆-Fettalkohol aufgegeben, wobei die Reaktionsteilnehmer eine Temperatur von 65°C aufweisen. Die Säurezahl des Gemisches beträgt 117 mg KOH/g. Im Reaktor (1) herrscht eine Temperatur von 250°C, als Inertgas wird Stickstoff durch die Leitung (6) zugeführt. Pro Stunde werden 6 l Flüssigkeit durch die Düse (3) mit Tröpfchengrößen im Bereich von 0,01 bis 1,0 mm in die Sprühzone (4) hinein versprüht. Nach 3 Stunden ist eine praktisch vollständige Veresterung erreicht und das Wachsester-Produkt weist eine Säurezahl von 0,09, eine Verseifungszahl von 135 mg KOH/g und ein Molekulargewicht von 422 auf. Dieser Wachsester wird in den nachfolgend beschriebenen Beispielen verwendet.

### Beispiel 1:

Im Labor wird die Hydrierung ähnlich Figur 3 durchgeführt, wobei 400 ml Hydrierkatalysator in einem Rohrreaktor ein Festbett von 30 mm Durchmesser und 200 mm Höhe bilden. Der Katalysator vom Typ T 4489 A (Hersteller: Süd-Chemie, München) liegt in Tabletten von 3 mm x 3 mm vor. Der Reaktor weist einen elektrischen Heizmantel auf, man arbeitet bei 230°C, einem Druck von 300 bar, einem Wachsester-Durchsatz von 750 ml/h und einer H₂-Zufuhr von 3,185 Nm³/h.

Das hydrierte Produkt, das ohne Kreislaufführung gewonnen wird, besteht aus 96,6 Gew.-% Fettalkohol und 3,4 Gew.-% Wachsester. Durch destillative Trennung erhält man als Kopfprodukt reinen Fettalkohol und als Sumpfprodukt Fettalkohol mit Wachsester. Dieses Sumpfprodukt kann in einer Anlage (W) zur Erzeugung von Wachsester verwendet werden, es hat eine Verseifungszahl von 4,6 und einen Kohlenwasserstoffgehalt von 0,75 Gew.-%.

### Beispiel 2:

Der bereits für Beispiel 1 verwendete Wachsester wird nunmehr in flüssiger Phase chargenweise mit darin verrührtem Katalysator hydriert, wobei man als Reaktionsgefäß einen 2 l fassenden Rührautoklaven verwendet. 20 g pulverförmiger Katalysator des Typs G 99 B-13 (Süd-Chemie) werden für 400 g Wachsester eingesetzt. Die H₂-Zufuhr erfolgt durch einen Hochdruck-Kompressor. Im Autoklaven wird die Temperatur auf 298°C und der Druck auf 300 bar gehalten, wobei mit 1000 Umdrehungen/min gerührt wird. In der nachfolgenden Tabelle sind die Ergebnisse der Hydrierung in Abhängigkeit von der Reaktionsdauer T angegeben, dazu die Verseifungszahl VZ der Flüssigkeit im Autoklaven in mg KOH/g und der Umsatz U des Wachsesters in Gew.-%.

| T(min) | 0 | 2 | 4 | 8 | 10 | 13 | 15 | 60 |
|---|---|---|---|---|---|---|---|---|
| VZ | 135 | 45,4 | 19,8 | 7,15 | 6,7 | 3,9 | 3,3 | 1,4 |
| U | 0 | 66,6 | 85,4 | 94,70 | 95,1 | 97,1 | 97,6 | 99,0 |

Die Säurezahl im Endprodukt ist o,08, und der Gehalt an Kohlenwasserstoffen liegt bei 0,5 Gew.-%.

## Patentansprüche

1. Verfahren zum Erzeugen von Fettalkohol aus einem mindestens eine Fettsäure enthaltenden flüssigen Ausgangsgemisch, wobei die Fettsäure pro Molekül 6 bis 30 C-Atome enthält, dadurch gekennzeichnet, daß das flüssige Ausgangsgemisch pro Mol Fettsäure 1,0 bis 1,5 Mol Fettalkohol aufweist, wobei der Fettalkohol pro Molekül 6 bis 30 C-Atome enthält, daß man aus dem flüssigen Ausgangsgemisch bei Temperaturen im Bereich von etwa 120 bis 320°C ein zu mindestens 50 Gew.-% aus Wachsester bestehendes flüssiges Zwischenprodukt erzeugt, wobei man das flüssige Ausgangsgemisch durch mindestens eine Rührzone leitet und wachsesterhaltiges flüssiges Gemisch in mindestens eine Sprühzone hinein versprüht, in der Sprühzone eine wasserdampfhaltige Atmosphäre erzeugt und die wasserdampfhaltige Atmosphäre mindestens teilweise entfernt, daß man das zu mindestens 50 Gew.-% aus Wachsester bestehende Zwischenprodukt hydriert und ein zu mindestens 75 Gew.-% aus Fettalkohol bestehendes Rohprodukt erzeugt, daß man aus dem Rohprodukt einen fettalkoholhaltigen Teilstrom abtrennt und den Teilstrom dem flüssigen Ausgangsgemisch zugibt.

2. Verfahren nach Anspruch 1, dadurch gekennzeichnet, daß das zu mindestens 50 Gew.-% aus Wachsester bestehende Zwischenprodukt ohne Verwenden eines feststoffhaltigen Katalysators erzeugt wird.

3. Verfahren nach Anspruch 1 oder 2, dadurch gekennzeichnet, daß man beim Erzeugen des Zwischenprodukts ein Inertgas in die Rührzone leitet und wasserdampfhaltiges Inertgas aus dem Gemisch ableitet.

4. Verfahren nach einem der Ansprüche 1 bis 3, dadurch gekennzeichnet, daß man im Gemisch einen Überschuß an Fettalkohol aufrechterhält und ein praktisch säurefreies Zwischenprodukt erzeugt.

5. Verfahren nach Anspruch 1 oder einem der folgenden, dadurch gekennzeichnet, daß man das Zwischenprodukt in der flüssigen Phase unter Zugabe von Wasserstoff und unter Zumischen eines feinkörnigen Katalysators hydriert.

6. Verfahren nach einem der Ansprüche 1 bis 4, dadurch gekennzeichnet, daß man das Zwischenprodukt zum Hydrieren zusammen mit Wasserstoff über einen im Festbett angeordneten Katalysator leitet.

7. Verfahren nach Anspruch 6, dadurch gekennzeichnet, daß man die Festbett-Hydrierung adiabatisch durchführt.

## Claims

1. A method for producing fatty alcohol from a liquid starting mixture containing at least one fatty acid, the fatty acid containing 6 to 30 C atoms per molecule, characterised in that the liquid starting mixture contains 1.0 to 1.5 moles fatty alcohol per mol fatty acid, the fatty alcohol containing 6 to 30 C atoms per molecule, that a liquid intermediate product consisting of at least 50% by weight wax ester is produced from the liquid starting mixture at temperatures in the range from about 120 to 320°C, the liquid starting mixture being passed through at least one stirring zone and liquid mixture containing wax ester is sprayed into at least one spraying zone, an atmosphere containing water vapour is produced in the spraying zone and the atmosphere containing water vapour is at least partially removed, that the intermediate product consisting of at least 50% by weight wax ester is hydrogenated and a raw product consisting of at least 75% by weight fatty alcohol is produced, that a partial stream containing fatty alcohol is separated out from the raw product and the partial stream is added to the liquid starting mixture.

2. A method according to Claim 1, characterised in that the intermediate product consisting of at least 50% by weight wax ester is produced without using a solids-containing catalyst.

3. A method according to Claim 1 or 2, characterised in that in producing the intermediate product an inert gas is passed into the stirring zone and inert gas containing water vapour is removed from the mixture.

4. A method according to one of Claims 1 to 3, characterised in that an excess of fatty alcohol is maintained in the mixture and an intermediate product which is practically acid-free is produced.

5. A method according to Claim 1 or one of the following claims, characterised in that the intermediate product is hydrogenated in the liquid phase with the addition of hydrogen and the admixing of a fine-grained catalyst.

6. A method according to one of Claims 1 to 4, characterised in that the intermediate product for hydrogenation is passed together with hydrogen over a catalyst arranged in a fixed bed.

7. A method according to Claim 6, characterised in that the fixed-bed hydrogenation is performed adiabatically.

## Revendications

1. Procédé pour produire un alcool gras à partir d'un mélange liquide de départ contenant au moins un acide gras, l'acide gras contenant par molécule 6 à 30 atomes de carbone, caractérisé en ce que le mélange liquide de départ comporte par mole d'acide gras 1,0 à 1,5 moles d'alcool gras, l'alcool gras contenant par molécule de 6 à 30 atomes de carbone ; que, à partir du mélange liquide de départ, à des températures comprises dans l'intervalle d'environ 120 à 320°C, on produit un produit intermédiaire liquide, constitué d'au moins 50 % en poids d'un ester creux, en faisant passer le mélange liquide de départ dans au moins une zone d'agitation, et en pulvérisant le mélange liquide, contenant l'ester cireux, dans au moins une zone de pulvérisation, en produisant dans la zone de pulvérisation une atmosphère contenant de la vapeur d'eau et en éliminant au moins partiellement l'atmosphère contenant de la vapeur d'eau ; que l'on hydrogène le produit intermédiaire, constitué d'au moins 50 % en poids d'un ester cireux, et on produit un produit brut, constitué d'au moins 75 % en poids d'un alcool gras ; que l'on sépare du produit brut un courant partiel contenant l'alcool gras, et que l'on ajoute le courant partiel au mélange liquide de départ.

2. Procédé selon la revendication 1, caractérisé en ce que le produit intermédiaire constitué d'au moins 50 % en poids d'ester cireux est produit sans utilisation d'un catalyseur contenant une matière solide.

3. Procédé selon la revendication 1 ou 2, caractérisé en ce que, lors de la production du produit intermédiaire, on introduit un gaz inerte dans la zone d'agitation et on évacue du mélange un gaz inerte contenant de la vapeur d'eau.

4. Procédé selon l'une des revendications 1 à 3, caractérisé en ce qu'on maintient dans le mélange un excès d'alcool gras, et que l'on produit un produit intermédiaire pratiquement sans acide.

5. Procédé selon la revendication 1 ou l'une des revendications suivantes, caractérisé en ce qu'on hydrogène le produit intermédiaire en phase liquide par addition d'hydrogène, tout en y mélangeant un catalyseur à fine granulométrie.

6. Procédé selon l'une des revendications 1 à 4, caractérisé en ce que le produit intermédiaire est, pour l'opération d'hydrogénation, envoyé en même temps que de l'hydrogène sur un catalyseur disposé en lit fixe.

7. Procédé selon la revendication 6, caractérisé en ce que l'hydrogénation en lit fixe est mis en oeuvre dans des conditions adiabatiques.
